# EUROPÄISCHE PATENTSCHRIFT

(11) **EP 1 613 748 B1**
(45) Veröffentlichungstag und Bekanntmachung des Hinweises auf die Patenterteilung: **15.04.2009**
(21) Anmeldenummer: 04713857.3
(22) Anmeldetag: 24.02.2004
(51) Int. Cl.: C12N 9/88

(54) **R-HYDROXYNITRILLYASEN MIT VERBESSERTER SUBSTRATAKZEPTANZ UND DEREN VERWENDUNG**
R-HYDROXYNITRILLYASES HAVING IMPROVED SUBSTRATE TOLERANCE AND THE USE THEREOF
R-HYDROXYNITRILLYASES A COMPATIBILITE AMELIOREE AVEC LE SUBSTRAT ET UTILISATION DESDITES SUBSTANCES

(30) Priorität: 20.03.2003 AT 4472003
(43) Veröffentlichungstag der Anmeldung: 11.01.2006
(73) Patentinhaber: DSM Fine Chemicals Austria Nfg GmbH & Co KG, 4021 Linz (AT)
(72) Erfinder: SKRANC, Wolfgang, A-1220 Wien (AT); GLIEDER, Anton, A-8200 Gleisdorf (AT); GRUBER, Karl, A-8042 Graz (AT); WEIS, Roland, A-8010 Graz (AT); LUITEN, Ruud, N-2317 KR Leiden (NL)
(74) Vertreter: Habets, Winand
(86) Internationale Anmeldenummer: PCT/EP2004/001778
(87) Internationale Veröffentlichungsnummer: WO 2004/083424

(56) Entgegenhaltungen:
- EP-A- 0 969 095
- EP-A- 1 223 220
- DREVENY INGRID ET AL: "The active site of hydroxynitrile lyase from Prunus amygdalus: Modeling studies provide new insights into the mechanism of cyanogenesis" PROTEIN SCIENCE, Bd. 11, Nr. 2, Februar 2002 (2002-02), Seiten 292-300, XP002281486 ISSN: 0961-8368
- DREVENY INGRID ET AL: "The hydroxynitrile lyase from almond: A lyase that looks like an oxidoreductase" STRUCTURE (CAMBRIDGE), Bd. 9, Nr. 9, September 2001 (2001-09), Seiten 803-815, XP002281487 ISSN: 0969-2126
- GRIENGL H ET AL: "The synthesis of chiral cyanohydrins by oxynitrilases" TRENDS IN BIOTECHNOLOGY, ELSEVIER PUBLICATIONS, CAMBRIDGE, GB, Bd. 18, Juni 2000 (2000-06), Seiten 252-256, XP002249789 ISSN: 0167-7799
- GLIEDER ANTON ET AL: "Comprehensive step-by-step engineering of an (R)-hydroxynitrile lyase for large-scale asymmetric synthesis." ANGEWANDTE CHEMIE (INTERNATIONAL ED. IN ENGLISH) GERMANY 13 OCT 2003, Bd. 42, Nr. 39, 13. Oktober 2003 (2003-10-13), Seiten 4815-4818, XP002281488 ISSN: 0570-0833

## Beschreibung

Biokatalytische Verfahren haben für die chemische Industrie eine hohe Bedeutung erlangt. Die Durchführung chemischer Reaktionen unter Zuhilfenahme biologischer Katalysatoren ist dabei vor allem in solchen Anwendungsgebieten von Interesse, in denen die oft vorhandene Eigenschaft von Enzymen, bei chemischen Reaktionen mit chiralen oder prochiralen Komponenten eines der beiden Enantiomeren bevorzugt umzusetzen oder zu bilden, genutzt werden kann.

Wesentliche Voraussetzungen für die Nutzung dieser günstigen Eigenschaften von Enzymen sind ihre Verfügbarkeit in technisch benötigten Mengen und eine ausreichend hohe Reaktivität, sowie Stabilität unter den realen Bedingungen eines technischen Verfahrens.

Eine besonders interessante Klasse von chiralen chemischen Verbindungen sind Cyanhydrine. Cyanhydrine sind beispielsweise bei der Synthese von α-Hydroxysäuren, α-Hydroxyketonen, β-Aminoalkoholen, die zur Gewinnung biologisch wirksamer Stoffe, z.B. pharmazeutischer Wirkstoffe, Vitamine oder pyrethroider Verbindungen Verwendung finden, von Bedeutung.

Die Herstellung dieser Cyanhydrine erfolgt durch Addition von Blausäure an die Carbonylgruppe eines Ketons oder Aldehyds.

Die technische Herstellung von chiralen Verbindungen, wie etwa (S)-Cyanhydrinen, konnte durch die Erschließung des Enzyms (S)-Hydroxynitril Lyase aus *Hevea brasiliensis* realisiert werden und ist beispielsweise in WO 97/03204, EP 0 951561 und EP 0 927 766 beschrieben.

Es gibt jedoch eine Vielfalt an interessanten chemischen Verbindungen, bei welchen die R-Enantiomeren für technische Anwendungen von Bedeutung sind. Bisher wurden lediglich Verfahren zur Herstellung einer Reihe von Produkten beschrieben, die nur im Labormaßstab eingesetzt werden können (z.B.: EP 0 276 375, EP 0 326 063, EP 0 547 655). Dabei wurden vorwiegend Enzympräparationen eingesetzt, die aus Pflanzen der Familie Rosaceae, beispielsweise aus den Kernen von Mandeln (*Prunus amygdalus*) gewonnen wurden. Weitere bisher eingesetzte *R*-HNLs sind beispielsweise solche aus Leinsamenkeimlingen (*Linum usitatissimum; Lu*HNL), die als erstes Gen einer *R*-HNL kloniert und in *E*. *Coli* und *Pichia pastoris* expremiert wurden oder R-HNL aus *Phlebodium aureurum.*

Technische Anwendungen im größeren Maßstab sind bislang nicht realisiert worden. Die wesentliche Ursache dafür ist, dass Enzympräparationen aus Pflanzen der Familie *Rosaceae* mit Hydroxynitrillyase(HNL)-Aktivität oder solche aus Leinsamenkeimlingen bisher nicht in ausreichenden Mengen und zu vertretbaren Kosten verfügbar waren und zudem eine zu geringe Stabilität bei tiefen pH-Werten zeigten.

Um Produkte mit hoher optischer Reinheit zu erhalten werden in der Literatur tiefe Temperaturen (zB.Persson et al; Enzyme and Microbial Technology 30(7), 916-923; 2002), ein pH-Wert unter 4 (zB. Kragl et al; Annals of the New York Academy of Science; 613 (enzyme Eng. 10), 167-75, 1990), sowie die Verwendung von 2-Phasensystemen (bspw. EP 0 547 655) oder von Emulsionen (zB. EP 1 238 094) als vorteilhafte Reaktionsparameter beschrieben.

Unglücklicherweise haben die meisten *R*-HNLs bei einem pH-Wert unter 4 Halbwertszeiten von weniger als einer Stunde.

Aus EP1223220 A1 sind rekombinante Enzyme, die durch Klonierung eines Gens aus *Prununs amygdalus,* das für ein *R*-HNL lsoenzym , beispielsweise für das lsoenzym 5 (*P*aHNL5) kodiert, sowie durch heterologe Expression beispielsweise in *Pichia pastoris* hergestellt werden, beschrieben, die sich, wie aus den Beispielen hervorgeht, durch eine wesentlich erhöhte Stabilität bei tiefen pH-Werten im Vergleich zu den anderen bekannten *R*-HNLs auszeichnet.

Als Nachteil wurde eine nicht zufriedenstellende Substratakzeptanz festgestellt, da die Umsetzung einiger Substrate, in Gegenwart von beispielsweise rekombinanter *Pa*HNL5 mit deutlich geringerer Reaktionsgeschwindigkeit als in Gegenwart von käuflichen pflanzlichen, nativen (*R*)-HNL Präparationen aus Mandelkernen erfolgt.

Aufgabe der Erfindung war es daher, *R*-Hydroxynitrillyasen aus der Familie *Rosaceae* bereitzustellen, die erstens für technische Umsetzungen im industriellen Maßstab in ausreichendem Maße und kostengünstig zur Verfügung gestellt werden können und die eine verbesserte Substratakzeptanz, sowie eine erhöhte Stabilität auf weisen.

Gegenstand der Erfindung sind demnach *R*-Hydroxynitrillyasen mit einer verbesserten Substratakzeptanz und erhöhter Stabilität, die dadurch gekennzeichnet sind, dass im aktiven Zentrum der *R*-Hydroxynitrillyasen entweder
ein Alaninrest durch Glycin, substituiert ist.

Die erfindungsgemäßen *R*-HNLs sind Mutanten von *R*-Hydroxynitrillyasen aus *Prunus amygdalys* (*PaHNL*) oder *Prunus serotina* (*Ps*HNL).

Als native. *R*-HNLs werden bevorzugt *R*-HNLs aus *Prunus amygdalys* (*P*aHNL), *Prunus domestica* (*P*dHNL) oder aus *Prunus serotina* (*Ps*HNL) eingesetzt.

Bevorzugte rekombinante *R*-HNLs sind die in EP 1223220 beschriebenen rekombinanten *R*-HNLs *Pa*HNL1 bis *P*aHNL5, besonders bevorzugt ist die rekombinante *P*aHNL5.

Die Herstellung der erfindungsgemäßen *R*-HNLs erfolgt durch ortsspezifische Mutagenese, beispielsweise mittels Quick Change (XL) Site Directed Mutagenesis Kit, Quick Change Multi Site Directed Mutagenese Kit (Fa. Stratagene), sowie Kits von Invitrogen oder Promega u.s.w. nach Anleitung des Herstellers oder gemäß anderen üblichen Methoden, wie etwa in Current Protocols in Molecular Biology, Ausubel et al., 2003 beschrieben.

Site Directed Mutagenesis Kits sind gebrauchsfertige Systeme zur Herstellung von spezifischen Mutanten und werden beispielsweise von der Fa. Stratagene Cloning Systems, La Jolla, CA (USA) kommerziell vertrieben.

Bei der ortsspezifische Mutagenese wird erfindungsgemäß im aktiven Zentrum der R-HNL ein Alaninrest durch Glycin substituiert.

Zur Umsetzung von großen Substraten, wie etwa von aromatischen Aldehyden oder Ketonen mit sperrigen Resten, bzw. mit Substituenten in ortho- oder meta-Stellung, oder von sperrigen aliphatischen Aldehyden oder Ketonen, wird bevorzugt ein Alanin gegen Glycin ausgetauscht.

Besonders bevorzugt sind Mutanten der beispielsweise aus EP 1223220 bekannten rekombinanten *R*-Hydroxynitirillyasen *Pa*HNL1 -*Pa*HNL5.

Insbesondere bevorzugt sind Mutanten der rekombinanten *R*-Hydroxynitrillyase *Pa*HNL5, bei welcher im aktiven Zentrum der Alaninrest in Position 111 durch Glycin substituiert ist.

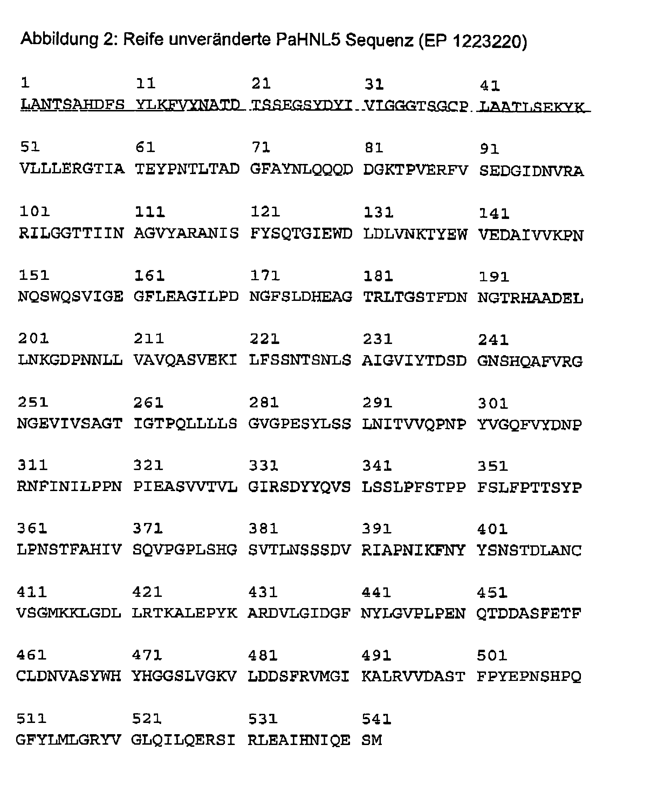

Die Nummerierungen gehen von den entsprechenden Positionen in der reifen unveränderten rekombinanten *R*-Hydroxynitrillyase *Pa*HNL5 aus Abbildung 2 die Positionen können sich jedoch durch die oben angeführten Änderungen der der Sequenz, wie etwa Verkürzung oder Verlängerung der Sequenz, dementsprechend verschieben.

Anschließend erfolgt die sekretorische Expression in geeigneten Mikroorganismen, wie etwa in *Pichia pastoris, Saccharomyces cerevisiae* oder *Escherichia coli, Bacillus subtilis, Klyveromyces lactis, Aspergillus niger, Penicillium chrysogenum, Pichia methanolica, Pichia polymorpha, Phichia anomala, Schizosaccharomyces pombe,* u.s.w..

Die Aufreinigung der resultierenden erfindungsgemäßen R-HNL-Mutanten erfolgt nach Standartmethoden, beispielsweise analog Dreveny et al; Structure (Cambridge; MA, United States) 9(9), 803-815; 2001.

Die erfindungsgemäßen R-HNL-Mutanten eignen sich zur Herstellung von enantiomerenreinen *R*-oder *S*-Cyanhydrinen in gegenüber dem Stand der Technik erhöhter Umsatzgeschwindigkeit, wobei sich die erfindungsgemäßen R-HNL-Mutanten auch durch eine hohe pH-Stabilität bei niedrigen pH-Werten auszeichnen.

Ein weiterer Gegenstand der Erfindung ist demnach die Verwendung der erfindungsgemäßen R-HNL-Mutanten zur Herstellung von enantiomerenreinen R-oder S-Cyanhydrinen.

Insbesondere werden die erfindungsgemäßen *R*-HNL-Mutanten bei aliphatischen und aromatischen Aldehyden und Ketonen als Substrate eingesetzt.

Unter Aldehyden sind dabei aliphatische, aromatische oder heteroaromatische Aldehyde zu verstehen. Unter aliphatischen Aldehyden sind dabei gesättigte oder ungesättigte, aliphatische, geradkettige, verzweigte oder cyclische Aldehyde zu verstehen. Bevorzugte aliphatische Aldehyde sind geradkettige oder verzweigte Aldehyde mit insbesondere 2 bis 30 C-Atomen, bevorzugt von 4 bis 18 C-Atomen, die gesättigt oder ein- oder mehrfach ungesättigt sind. Der Aldehyd kann dabei sowohl C-C-Doppelbindungen als auch C-C-Dreifachbindungen aufweisen. Die aliphatischen, aromatischen oder heteroaromatischen Aldehyde können weiters unsubstituiert oder durch unter den Reaktionsbedingungen inerte Gruppen, beispielsweise durch gegebenenfalls substituierte Aryl- oder Heteroarylgruppen, wie Phenyl-, Phenoxy- oder Indolylgruppen, durch Halogen-, Hydroxy-, Hydroxy-C₁-C₅-Alkyl, C₁-C₅-Alkoxy-, C₁-C₅-Alkylthio-, Ether-, Alkohol-, Carbonsäureester-, Nitro- oder Azidogruppen substituiert sein.

Beispiele für bevorzugte aliphatische Aldehyde sind Butanal, 2-Butenal, 3-Phenylpropanal, Hydroxypivalaldehyd u.s.w..

Beispiele für aromatische oder heteroaromatische Aldehyde sind Benzaldezyd bzw. verschieden substituierte Benzaldehyde wie etwa 2-Chlorbenzaldehyd, 3-Chlorbenzaldehyd, 4-Chlorbenzaldehyd, 3,4-Difluorbenzaldehyd, 3-Phenoxybenzaldehyd, 4-Fluor-3-phenoxybenzaldehyd, Hydroxybenzaldehyde, Methoxybenzaldehyde, weiters Furfural, Methylfurfural, Anthracen-9-carbaldehyd, Furan-3-carbaldehyd, Indol-3-carbaldehyd, Naphthalin-1-carbaldehyd, Phthaldialdehyd, Pyrazol-3-carbaldehyd, Pyrrol-2-carbaldehyd, Thiophen-2-carbaldehyd, Isophthalaldehyd oder Pyridinaldehyde, Thienylaldehyde u.s.w.

Ketone sind aliphatische, aromatische oder heteroaromatische Ketone, bei denen das Carbonylkohlenstoffatom ungleich substituiert ist. Unter aliphatischen Ketonen sind gesättigte oder ungesättigte, geradkettige, verzweigte oder cyclische Ketone zu verstehen. Die Ketone können gesättigt oder ein- oder mehrfach ungesättigt sein. Sie können unsubstituiert, oder durch unter Reaktionsbedingungen inerte Gruppen, beispielsweise durch gegebenenfalls substituierte Aryl- oder Heteroarylgruppen wie Phenyl- oder Inolylgruppen, durch Halogen-, Ether-, Alkohol-, Carbonsäureester-, Nitro- oder Azidogruppen substituiert sein.

Beispiele für aromatische oder heteroaromatische Ketone sind Acetophenon, Indolylaceton u.s.w..

Aldehyde und Ketone, die sich erfindungsgemäß eignen, sind bekannt oder wie üblich herstellbar.

Die Substrate werden in Anwesenheit der erfindungsgemäßen HNLs mit einem Cyanidgruppendonor umgesetzt.

Als Cyanidgruppendonor kommen Blausäure, Alkali-Cyanide oder ein Cyanhydrin der allgemeinen Formel I

R₁R₂C(OH)(CN),

in Betracht. In der Formel I bedeuten R₁ und R₂ unabhängig voneinander Wasserstoff oder eine unsubstituierte Kohlenwasserstoffgruppe, oder R₁ und R₂ gemeinsam eine Alkylengruppe mit 4 oder 5 C-Atomen, wobei R₁ und R₂ nicht gleichzeitig Wasserstoff bedeuten. Die Kohlenwasserstoffgruppen sind aliphatische oder aromatische, bevorzugt aliphatische Gruppen. Bevorzugt bedeuten R₁ und R₂ Alkylgruppen mit 1 - 6 C-Atomen, ganz bevorzugt ist der Cyanidgruppendonor Acetoncyanhydrin.

Die Herstellung des Cyanidgruppendonors kann nach bekannten Verfahren erfolgen. Cyanhydrine, insbesonders Acetoncyanhydrin, sind auch käuflich zu erwerben. Bevorzugt wird Blausäure (HCN), KCN, NaCN, oder Acetoncyanhydrin, besonders bevorzugt Blausäure als Cyanidgruppendonor eingesetzt.

Die Blausäure kann dabei auch erst kurz vor der Reaktion aus einem ihrer Salze wie etwa NaCN oder KCN freigesetzt und in Substanz oder in gelöster Form dem Reaktionsgemisch zugegeben werden.

Die Umsetzung kann im organischen, wässrigen oder 2-Phasensystem oder in Emulsion, sowie in Substanz durchgeführt werden.

Als wässriges System wird eine wässrige, die erfindungsgemäße HNL enthaltende Lösung oder Pufferlösung verwendet. Beispiele dafür sind Na-Citratpuffer, Phosphatpuffer u.s.w.

Als organisches Verdünnungsmittel können mit Wasser nicht oder geringfügig mischbare aliphatische oder aromatische Kohlenwasserstoffe, die gegebenenfalls halogeniert sind, Alkohole, Ether oder Ester oder Gemische davon oder das Substrat selbst verwendet werden. Bevorzugt werden Methyl-tert. Butylether (MTBE), Diisopropylether, Dibutylether und Ethylacetat oder deren Gemisch eingesetzt.

Die erfindungsgemäßen HNLs können dabei entweder als solche oder immobilisiert in dem organischen Verdünnungsmittel vorliegen, die Umsetzung kann jedoch auch in einem Zweiphasensystem oder in Emulsion mit nicht-immobilisierter HNL erfolgen.

Die Umsetzung erfolgt dabei bei Temperaturen von -10°C bis +50°C, bevorzugt bei -5°C bis + 45°C.

Der pH-Wert des Reaktionsgemisches kann von 1.8 bis 7, bevorzugt von 2 bis 4 und besonders bevorzugt von 2.5 bis 3.5 betragen.

### Beispiel 1: Klonierung des pahnl4 Gens aus Prunus amygdalus

Es wurden genspezifische PCR Primer basierend auf der Sequenzhomologie des mdl4 Gens von *Prunus serotina* hergestellt:

Die Amplifikation erfolgte in einem 50 µL Ansatz mit 1,2 U "Hotstar" Taq DNA Polymerase (Qiagen, Hilden, Deutschland), mit 50 ng genomischer Mandel-DNA (isoliert aus Farmgold Mandelkernen Chargennummer L4532, Ernte 1999) als "Vorlage", je 10 pMol Primer mandlp2f (oBT2204) und mandlp5r (oBT2206), 5 µL dNTP (je 2 mM) Mix, alles in 1x PCR Puffer entsprechend dem Manual des "Hotstar Kit" (Qiagen, Hilden, Deutschland), beginnend mit einem 15 minütigen Denaturierungsschritt bei 95 DEG C, gefolgt von 10 Zyklen (1 min 94 DEG C, 1 min 45 DEG C; 1 min 72 DEG C) zur Voramplifikation, 20 weiteren Zyklen (1 min 94 DEG C, 1 min 68 DEG C, 1 min 72 DEG C) zur Amplifikation spezifischer Produkte und einer abschließenden Inkubation für 5 min bei 72 DEG C. Durch diese PCR wurde ein DNA Fragment in der Grösse von ca. 2,2 kb (festgestellt durch Analyse mittels Agrose-Gelelektrophorese) erhalten. Dieses PCR Produkt wurde mittels "Qiaquick Kit" (Qiagen, Hilden, Deutschland) entsprechend dem beigefügten Manual aus einem Agarosegel gereinigt, über die *Eco*RI Schnittstellen in den Kloniervektor pBSSK(-) einkloniert und unter Verwendung des "Dye Deoxy Terminator Cycle Sequencing" Kits (Applied Biosystems Inc., Forster City, CA, USA) nach der Strategie des "Primer walkings" sequenziert. Aus dem 5'- und 3'- Bereich der DNA Sequenz wurden neue PCR primer abgeleitet und für eine erneute PCR eingesetzt. Dabei wurde folgender Reaktionsansatz gewählt: 20 ng genomische DNA, je 10 pMol der beiden primer pamhnl4a (oBT2544) und pamhnl4e (oBT2543), 2 µL dNTP Mix (je 5 mM), 1 x Hotstar PCR Puffer und 1,2 U Hotstar DNA Polymerase (Qiagen, Hilden, D). Die Amplifikation erfolgte nach einem 15 minütigen Schritt bei 95 DEG C mit 30 Zyklen (1 min 94 DEG C, 30 sec 60 DEG C, 2 min 72 DEG C) und 15 min bei 72 DEG C. Das PCR Produkt wurde 2 mal über Qiaquick (Qiagen, Hilden, D) Säulchen gereinigt und direkt sequenziert, um Sequenzfehler von klonierten PCR Produkten zu vermeiden. In der erhaltenen DNA Sequenz des insgesamt 2232 Basenpaare langen PCR Fragments wurden die E-xons identifiziert und aus der kodierenden Sequenz wurde die Proteinsequenz des Isoenzyms *Pa*HNL4 abgeleitet.
oBT2204 mandlp2f: 5'-ACTACGAATTCGACCATGGAGAAATCAAC-3'
oBT2206 mandlp5r: 5'-CACTGGAATTCAAAGAGCAACACTTATCCACGG-3'
oBT2543 pamhnl4e: 5'-AAGAGGAACACTTAGCCACG-3'
oBT2544 pamhnl4a: 5'-CAACAATGTCCGCTGTAGTG-3'

### Beispiel 2: Austausch der Signalsequenz

Als N-Terminus des berechneten reifen Proteins (sekretiertes Protein nach Abspaltung des Signalpeptids und der zusätzlichen GluAlaGluAla Sequenz) wurden 2 Varianten gewählt, um eine Akkumulation von unvollständig prozessiertem Enzym im Zellinneren zu vermeiden:
A) Leucin als N-terminale Aminosäure, wie sie auch in der Wildtyp Sequenz vorkommt und die nach der N-Endrule als primär destabilisierende Aminosäure gilt (Varshavsky et al., Proceedings of the National Academy of Science of the United States of America, 93(22), 12142-12149, 1996).
B) Glutamin (Mutation L1Q) als N-terminale Aminosäure, die als tertiär destabilisierende Aminosäure beschrieben ist (Varshavsky et al., 1996).

### PCR I:

Die Signalsequenz des alpha mating Faktors von Saccharomyces *cerevisiae* wurde von der Vorlage pPICZB (Invitrogen Inc, San Diego, Ca, Cat. Nr. V19520) hochamplifiziert. Die PCR primer wurden so erstellt, dass die *Eco*RI Schnittstelle vom Invitrogen Plasmid am 3' Ende der Signalsequenz zerstört wurde. So wurde die Möglichkeit geschaffen, das gesamte Genkonstrukt inklusive der Signalsequenz über *Eco*RI Schnittstellen in verschiedene *Pichia* Expressionsvektoren zu klonieren. Dazu wurden die Primerpaare alpha11/alpha21a bzw alpha11/alpha21aQ verwendet. Die Primer alpha 11 und hnl5α21 enthalten eine *Eco*RI Schnittstelle. Die Primer alpha21a bzw. alpha21aQ enthielten auch einen DNA Sequenzbereich, der für das 5'Ende des reifen Isoenzyms *Pa*HNL5 kodiert. Der Primer alpha21aQ enthielt eine Sequenzänderung, die zur Mutation L1Q am N-Terminus des erwarteten reifen sekretierten Proteins führt. Am Ende der alpha Faktor Signalsequenz befand sich eine Kex2 Spaltstelle und eine von Ste13 prozessierte GluAlaGluAla-Sequenz.

Die PCR wurde in einem 50 µL Ansatz (10 ng Vorlage, je 0,1 µM Primer, 0,2 mM dNTPs, 5 µL PCR Puffer, 1 U *Pwo* Polymerase von Roche) in einem Thermocycler von Applied Biosystems (Forster City, CA) durchgeführt. Nach einem Denaturierungsschritt für 2 min bei 94 °C erfolgte die Amplifikation in 30 Zyklen (30 sec 94 °C, 60 sec 62 °C, 1 min 30 sec 72 °C) und einem Abschlussschritt für 7 min bei 72 °C.

### PCR II:

Das hnl5 Gen wurde aus dem Plasmid pHILDPaHNL5a (BT4256) mit den Primerpaaren hnl5α11/hn15α21 bzw. hnl5α11Q/hnl5α21 hochamplifiziert. Die Primer hnl5α11 und hnl5α11Q enthielten auch einen DNA Sequenzbereich, der dem 3' Ende des Fragments mit der alpha Faktor Signalssequenz (siehe oben) entsprach. Der Primer hnl5α21 enthielt eine *Eco*RI Schnittstelle.

Die PCR wurde in einem 50 µL Ansatz (10 ng Vorlage, je 0,1 µM Primer, 0,2 mM dNTPs, 5 µL PCR Puffer, 1 U *Pwo* Polymerase von Roche) in einem Thermocycler von Applied Biosystems durchgeführt. Nach einem Denaturierungsschritt für 2 min bei 94 °C erfolgte die Amplifikation in 30 Zyklen (30 sec 94 °C, 60 sec 65 °C, 3 min 30 sec 72 °C) und einem Abschlussschritt für 7 min bei 72 °C.

### Overlap extension: .

Je 3 µL der beiden Produkte aus PCR I und PCR II wurden als Vorlage und zugleich als Primer für eine Vervollständigung zu einem zusammenhängenden Produkt eingesetzt. Die Extension erfolgte in einem 45 µL Ansatz mit 5 µL *Pwo* PCR Puffer, 0,2 mM dNTPs und 1 Unit *Pwo* Polymerase (Roche, Mannheim, D). Der Ansatz wurde 2 min bei 94 °C erhitzt und dann mit 10 Zyklen zu 30 sec bei 94 °C und 3 min bei 72 °C im Thermocycler inkubiert.

### PCR III:

Zur Amplifikation des Produktes aus der Overlap Extension wurden die Primer alpha11 und hnl5α**2**1 verwendet. Zum Ansatz der Overlap extension PCR wurden 5 µL Primermix (3 µL Wasser und je 1 µL Primer alpha11, bzw. hnl5α21, wobei die Konzentration der Primer 5 µM war) zugesetzt und das Produkt mit 20 Zyklen (30 sec 94 °C, 45 sec 62 °C, 4 min 72 °C) amplifiziert. Zum Abschluss erfolgte eine Inkubation bei 72 °C für 7 min. Das PCR Produkt wurde nach dem Qiaquick Reinigungsprotokoll von Qiagen (Hilden, D) gereinigt, mit *Eco*RI geschnitten und in den Vektor pHILD2 (Invitrogen, San Diego, Ca) kloniert.

### Primersequenzen:

oBT2835 alpha11: 5'- tcttcgaagaattcacgATGAGATTTCCTTCAATTTTTACTGC- 3'
oBT2841 alpha21a: 5'- gaagtattggcaagAGCTTCAGCCTCTCTTTTCTCG- 3'
oBT2843 alpha21aQ: 5'- gaagtattggcttgAGCTTCAGCCTCTCTTTTCTCG-3'
oBT2837 hn15α11: 5'- agagaggctgaagctCTTGCCAATACTTCTGCTCATG-3'
oBT2842 hnl5a11Q: 5'- agagaggctgaagctCAAGCCAATACTTCTGCTCATG-3'
oBT2838 hn15α21: 5'- atggtaccgaattcTCACATGGACTCTTGAATATTATGAATAG-3'
Extensionen sind kleingeschrieben.

Die resultierenden Plasmide wurden pHILDPaHNL5α (BT4338) bzw. pHILD*Pa*HNL5α_L1Q (BT4339) genannt. Die Transformation in *Pichia pastoris* erfolgte nach der Standardvorschrift von Invitrogen.

Je ca. 2000 Transformanten wurden mit sterilen Zahnstochern in deep well Kulturplatten beimpft und für das Screening nach aktiven Transformanten angezüchtet.

### Beispiel 3: Anzucht von Pichia pastoris Transformanten und Produktion von PaHNL5 Varianten

### a) Mikrokulturen in deep well plates

250 µL BM0,5G Medium (0,2 M Kaliumphosphat, pH6,0; 13,4 g /L Yeast Nitrogen Base, 5 g/L Glyzerin, 0,8 mg/L Biotin) in 2 mL deep well plates wurden mit Einzelkolonien von Transformanten beimpft und bei 28 °C und 340 rpm geschüttelt. Die Induktion der Expression über den AOX1 Promotor erfolgte durch Zugabe von 250 µL BMM2 Medium (0,2 M Kaliumphosphat, pH 6,0; 13,4 g/L Yeast Nitrogen Base, 20 mL /L Methanol, 0,8 mg/L Biotin) nach 60 - 70 Stunden. Nach 10, 24 und 48 Stunden erfolgten weitere Methanolgaben durch Zugabe von je 50 µL BMM10 (0,2 M Kaliumphosphat, pH6,0; 13,4 g/L Yeast Nitrogen Base, 100 mL/L Methanol, 0,8 mg/L Biotin).

Ca. 72 Stunden nach der Induktion wurden die Zellen abzentrifugiert und der Kulturüberstand direkt, verdünnt, oder aufkonzentriert durch Ultrafiltration über 30 kDa Ausschlussmembranen Vivaspin von Sartorius (Göttingen, D), zur Vermessung der Enzymaktivität eingesetzt.

### b) "Scale up" in Schüttelkolben

225 mL BM0,5G Medium (0,2 M Kaliumphosphat, pH6,0; 13,4 g /L Yeast Nitrogen Base, 5 g/L Glyzerin, 0,8 mg/L Biotin) in 2 Liter Schikanenkolben wurden mit einer großen Einzelkolonie beimpft und bei 28 °C und 120 rpm geschüttelt. Die Induktion der Expression über den AOX1 Promotor erfolgte durch Zugabe von 25 mL BMM10 Medium (0,2 M Kaliumphosphat, pH6,0; 13,4 g/L Yeast Nitrogen Base, 100 mUL Methanol, 0,8 mg/L Biotin) nach 60 - 70 Stunden. Nach 10, 24 und 48 Stunden erfolgten weitere Methanolgaben von 2,5 mL pro Schüttelkolben (250 mL).

Ca. 72 Stunden nach der Induktion wurden die Zellen abzentrifugiert und der Kulturüberstand direkt, verdünnt oder durch Ultrafiltration über 30 kDa Ausschlussmembranen aufkonzentriert zur Vermessung der Enzymaktivität eingesetzt.

### Beispiel 4: Ortsspezifische Mutagenese

10 ng des Expressionsplasmids pHILDPaHNL5α_L1Q (PaHNL5 mit alpha Faktor Signalsequenz) wurden als Vorlage für die Mutagenesereaktion mittels Quik Change XL Site Directed Mutagenesis Kit von Stratagene (Cat. # 200516) eingesetzt. Für die Reaktion wurden je 200ng der beiden Mutageneseprimer eingesetzt. Folgendes Temperaturprogramm wurde verwendet:
A) Denaturierung bei 95 °C für eine Minute
B) 18 Zyklen mit 50 sec bei 95 °C, 50 sec bei 60 °C und 20 min bei 68 °C
C) Extension für 7 min bei 68 °C

Die Vorlagen DNA wurde wie im Protokoll des Kits beschrieben mit *Dpn*I abverdaut und 2 µL des Ansatzes wurden wie beschrieben zur Transformation von ultrakompetenten *E. coli* XL10 Gold Zellen eingesetzt. Von den Transformanten wurde Plasmid DNA präpariert und sequenziert. Plasmide von Mutanten mit korrekter Sequenz im Bereich des kodierenden DNA Inserts wurden vermehrt und mit Hilfe der Standardarbeitsvorschrift von Invitrogen) in *Pichia pastoris* GS115 transformiert.

Mehrere hundert Histidin autotrophe *Pichia* Transformanten wurden wie oben beschrieben in deep well Platten kultiviert und die Aktivität der Kulturüberstände mit racemischem Mandelonitril in 96 well plates bestimmt. Klone mit der jeweils höchsten Enzymaktivität der einzelnen Mutanten wurden für Schüttelkolbenversuche ausgewählt. Die Enzymaktivität der Kulturüberstände wurde mit dem Substrat Mandelonitril, (DSM Fine Chemicals Linz, A) bestimmt.

Folgende Mutationen wurden durchgeführt:
A111G, A111L, A111V, F72A, L331A und L343A, sowie als Vergleichsversuch V317A und V317G

PCR Primer für die ortsspezifische Mutagenese:
oBT2966 oPaHNL5A111Gf:
   5'-GTGGCACGACCATAATCAATGGAGGCGTCTACGCCAGAGCTAAC-3'
oBT2967 oPaHNL5A111Gr:
   5'-GTTAGCTCTGGCGTAGACGCCTCCATTGATTATGGTCGTGCCAC-3'
oBT3080 oPaHNL5A111Lf
   5' GCACGACCATAATCAATGCTTGCGTCTACGCCAGAGCTAAC 3'
oBT3081 oPaHNL5A111Lr
   5' GTTAGCTCTGGCGTAGACGCAAGCATTGATTATGGTCGTGCCAC 3'
oBT3078 oPaHNL5A111Vf
   5' GTGGCACGACCATAATCAATGGTTGCGTCTACGCCAGAGCTAAC 3'
oBT3079 oPaHNL5A111Vr
   5' GTTAGCTCTGGCGTAGACGCAACCATTGATTATGGTCGTGCCAC 3'
oBT2983 oPaHNL5V317(A,G)f
   5' TCCAATTGAAGCCTCTGTTGSAACTGTTTTAGGCATTAGAAGTG 3'
oBT2984 oPaHNL5V317(A,G)r:
   5' CTAATGCCTAAAACAGTTSCAACAGAGGCTTCAATTGGATTTGG 3'
oBT3017 oPaHNL5F72Af:
   5' CACGTTGACTGCAGATGGGGCTGCATATAATCTGCAGCAACAAG 3'
oBT3018 oPaHNL5F72Ar:
   5' CTTGTTGCTGCAGATTATATGCAGCCCCATCTGCAGTCAACGTG 3'
oBT3019 oPaHNL5L343Af
   5' CCACTCCACCCTTTAGTGCTTTTCCTACAACATCTTACCCCCTC 3'
oBT3020 oPaHNL5L343Ar:
   5' AAGATGTTGTAGGAAAAGCACTAAAGGGTGGAGTGGAAAATGGC 3'
oBT3021 oPaHNL5L331Af:
   5' AGTGATTATTATCAAGTTTCTGCCTCAAGCTTGCCATTTTCCAC 3'
oBT3022 oPaHNL5L331Ar
   5' GGAAAATGGCAAGCTTGAGGCAGAAACTTGATAATAATCACTTC 3'
**S=G oder C**

### Beispiel 5: Sequenzkontrolle mittels "Kolonie PCR"

Zur Kontrolle, ob bei der *Pichia* Transformation oder während der Selektion des Stammes kein Fehler passiert ist wurde das integrierte mutierte HNL5 Gen mittels PCR amplifiziert und im Bereich der durchgeführten Mutation sequenziert.

Dazu wurde eine Einzelkolonie mit 0,5 mL Wasser suspendiert und 30 min. bei 95 °C gekocht. Dieser Ansatz wurde dann 1 min. bei 13 500 rpm in einer Tischzentrifuge zentrifugiert. 5 µL des Überstands wurden als Vorlage für die PCR Amplifikation des mutierten hnl5 Gens eingesetzt. Der Gesamtansatz mit einem Volumen von 50 µL beinhaltete ausserdem je 0,2 mM der beiden Primer oBT2907 und oBT2908a, 0,2 mM dNTPs, 1x Hotstar (Qiagen) PCR Puffer und 2,5 U Hotstar DNA Polymerase. Die 3 stufige PCR erfolgte in folgenden Schritten:
Einmal 95°C für 15 min, dann 30 Zyklen mit 30 Sekunden bei 94 °C, 1 min bei 55°C und 2,5 min bei 72 °C und zum Abschluss einmal 72 °C für 10 min.
3 µL dieses PCR Ansatzes wurden nach der Amplifikation als Vorlage zur Reamplifikation eingesetzt, um genügend Material für die DNA Sequenzierung zu erhalten. Die Reaktionsbedingungen waren gleich wie bei der Erstamplifikation. Das PCR Produkt aus der Reamplifikation wurde nach zweimaliger Reinigung nach der Qiaquick Methode von Qiagen zur Sequenzierung eingesetzt.

oBT2907: 5'- GCAAATGGCATTCTGACATCC -3'
oBT2908a: 5'- GACTGGTTCCAATTGACAAGC -3'

Transformanten, die die *Pa*HNL5 Mutanten exprimierten wurden isoliert und der Bereich der Mutation zur Kontrolle nach der Kolonie PCR Methode sequenziert.

In Tabelle 1 ist eine Übersicht zu den jeweiligen Expressionsstämmen der Mutanten dargestellt:

**Tabelle 1: eingeführte Änderungen im pahnl5 Gen**

| Signalsequenz | Pichia Klon-Name | Pichia Klon Nr | Plasmid für Pichia Transformation | AS-Austausch | DNA Sequenz |
|---|---|---|---|---|---|
| plant PaHNL5 signal | GS115pHILDPaHNL1a37 | BT2578 | BT4256 | | |
| Alpha factor | GS115pHILDPaHNL5alpha_G13 | BT2617 | BT4338 | | |
| Alpha factor | GS115pHILDPaHNL5alphaLlQ-H21 | BT2620 | BT4339 | L1Q | CTT -> CAA |
| Alpha factor | GS115pHILDPaHNL5alpha_ L1Q,A111G ( = spla8F9) | BT2621 | BT4345 | L1Q | CTT -> CAA |
| | | | | A111G | GCA -> GGA |
| Alpha factor | GS115pHILDPaHNL5alpha_ L1Q,V317A (= spA2.2) | BT2623 | BT4375 | L1Q | CTT -> CAA |
| | | | | V317A | GTA -> GCA |
| Alpha factor | GS115pHILDPaHNL5alpha_ L1Q,V317G (= spAa) | BT2624 | BT4376 | L1Q | CTT-> CAA |
| | | | | V317G | GTA -> GGA |
| Alpha factor | GS115pHILDPaHNL5alpha_ L1Q, F72A | BT2632 | BT4400 | L1Q | CTT-> CAA |
| | | | | F72A | TTT -> GCT |
| Alpha factor | GS115pHILDPaHNL5alpha_ L1Q,L331A | BT2633 | BT4401 | L1Q | CTT -> CAA |
| | | | | L331A | CTG -> GCC |
| Alpha factor | GS115pHILDFaHNLSalpha_ L1Q,L343A | BT2634 | BT4402 | L1Q | CTT -> CAA |
| | | | | L343A | CTT -> GCT |
| Alpha factor | GS115pHILDPaHNLSalpha_ L1Q, F72A, L331A | BT2635 | BT4403 | L1Q | GTT -> CAA |
| | | | | F72A | TTT -> GCT |
| | | | | L331A | CTG -> GCC |
| Alpha factor | GS115pHILDPaHNL5alpha_ L1Q, F72A, L331A, L343A | BT2636 | BT4404 | L1Q | CTT -> CAA |
| | | | | F72A | TTT -> GCT |
| | | | | L331A | CTG -> GCC |
| | | | | L343A | CTT -> GCT |
| Alpha factor | GS115pHILDPaHNL5alpha_ L1Q, A111L | BT2637 | BT4405 | L1Q | CTT -> CAA |
| | | | | A111L | GCA -> CTA |
| Alpha factor | GS115pHILDPaHNLSalpha_ L1Q, A111V | BT2638 | BT4406 | L1Q | CTT -> CAA |
| | | | | A111V | GCA -> GTT |
| | | | | | |

### Beispiel 6: Reinigung und Charakterisierung von Prunus amygdalus Enzymvarianten

Zur Bestimmung der spezifischen Aktivität der jeweiligen Mutanten mit verschiedenen Substraten wurden von den Expressionsklonen jeweils mehrere Schüttelkolbenkulturen durchgeführt. Der Kulturüberstand wurde durch Ultrafiltration (30 kDa Ausschlussgrösse) mit 20 ml Vivaspin PES Zentrifugationssäulchen von Sartorius (Göttingen, D) aufkonzentriert und anschließend chromatographisch gereinigt.

Vor der Reinigung wurde der aufkonzentrierte Kulturüberstand durch wiederholte Verdünnung und Konzentration mit Bindepuffer A (20 mM Citrat-Phosphat-Puffer, pH 5.5) in 30 kDa Ultrafiltrations Zentrifugationsmodulen (Vivaspin, Sartorius) mit dem salzarmen Bindepuffer A äquilibriert und dann auf einer ÄKTApurifier 10 FPLC Anlage von Amersham Biosciences UK Limited (Buckinghamshire, GB) über eine Anionentauscher Q-Sepharose Fast Flow (QFF) Säule mit einem Säulenvolumen von 10 mL gereinigt. Die Elution erfolgte mit Elutionspuffer B (20 mM Citrat-Phosphat-Puffer + 1 M NaCl, pH 5.5), wobei für die verschiedenen Varianten von *Pa*HNL5 aus heterologer Produktion mit *Pichia pastoris* folgendes Gradientenprofil verwendet wurde: Ein Säulenvolumen als Waschschritt erwies sich als ideal, um alle ungebundenen Proteinbestandteile auszuwaschen. Innerhalb eines halben Säulenvolumens wurde die Konzentration von Puffer B (Elutionspuffer: 20mM Citrat-Phosphat-Puffer, 1 M NaCI, pH 5.5) auf 4% gehoben, um in weiterer Folge auf 48% innerhalb eines weiteren Säulenvolumens gesteigert zu werden. Im nächsten Schritt wurde die Konzentration des Elutionspuffers B auf 70% erhöht, wobei hier 1 ½ Säulenvolumen gebraucht wurden.

Schlussendlich wurde die Konzentration innerhalb eines Säulenvolumens auf das Maximum von 100% gehoben, und abschließend für ein weiteres Säulenvolumen dort belassen (Waschschritt ohne Fraktionierung).

Von jenen Fraktionen, die nach Auswertung des Chromatogramms Protein enthalten sollten (abhängig von Peak-Lage), wurden mit dem Biorad (Hercules, Ca) Proteinassay (Bradford Methode) der Proteingehalt und mit dem Substrat Mandelonitril die Enzymaktivität bestimmt. Die 2-3 Fraktionen mit der höchsten Aktivität wurden gepoolt und für die Analyse der Enzymcharakteristika eingesetzt. Die Proteinkonzentration wurde mit einem Biorad (Hercules, Ca) Proteinassay (Bradford) durchgeführt. Als Standard zur Herstellung einer Eichgeraden diente native *Pa*HNL von Sigma (M-6782 Lot 41 H4016).

Die Kulturüberstande wurden über cross-flow-Filtration ∼20-fach konzentriert und anschließend chromatographisch gereinigt. Von den gereinigten Enzymen, wurden Proben entnommen und direkt auf ein Gel (Proteingel NuPAGE 4-12% Bis Gel 1 mm X 17 well; Invitrogen) aufgetragen, bzw. ∼500ng mit Endoglykosidase H (#P0702L, NEB,) deglykosyliert (nach enthaltener Vorschrift) und dann aufgetragen. Als Standard wurde "SeeBlue Plus2 Pre-Stained Standard" von Invitrogen (Carlsbad, USA) verwendet.

Zum Vergleich der Substratspezifitäten wurde die Proteinkonzentration der gereinigten Enzyme und der Proteingehalt im Kulturüberstand mit dem Biorad Proteinassay (Hercules, Ca) gemessen und die spezifischen Aktivitäten mit racemischem Mandelonitril fotometrisch bzw. mit 2-, 3- und 4-Chlorbenzaldehyd mittels GC verglichen:

Dazu wurden 15 mMol Substrat in 2,1mL tert-Butylmethylether (MTBE) gelöst. 0,25 mg der entsprechenden *Pa*HNL wurden mit 50 mM K2HPO4/Zitrat Puffer pH3,4 auf ein Endvolumen von 3,7 mL verdünnt, der Puffer erneut auf pH 3,4 eingestellt und dann in 20 mL Glasphiolen mit dem Substrat in MTBE vermischt. Die Lösung wurde auf 10°C gekühlt und 1,2 mL HCN mit einer Spritze zudosiert und am Magnetrührer zur Bildung einer Emulsion bei 10°C gerührt. Zu verschiedenen Zeitpunkten wurden Proben entnommen, mit Essigsäureanhydrid in Gegenwart von Pyridin und Dichlormethan derivatisiert und mittels GC an einer Cyclodextrinsäule (CP-Chirasil-Dex CB) oder mittels HPLC analysiert.

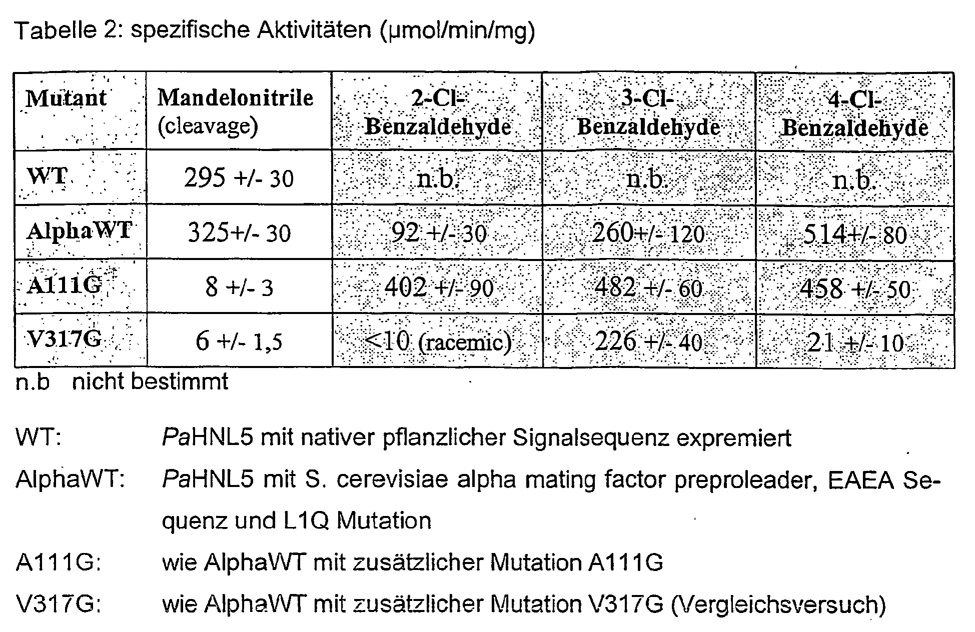

Die Messungen ergaben im Vergleich zu den rekombinanten Wildtyp WT und AlphaWT Isoenzymen *Pa*HNL5 eine ca. 3-5 fach höhere spezifische Aktivität der A111G Mutante mit dem Substrat (R)-2 Chlormandelonitril. Auch die Aktivität mit 3-Chlorbenzaldehyd war mit der A111G Mutante höher als mit AlphawT.

### Beispiel 7: Herstellung der Mutante A111G

Vom verbesserten Klon *Pichia pastoris* GS115 pHILDPaHNL5alpha_L1Q,A111G (=BT2621) wurde in einer Pilotfermentation eine ausreichende Menge an Enzym für Pilotumsetzungen hergestellt.

8 Schikanenkolben (2 L Weithals) mit je 250 ml BMG Medium (nach dem Standardprotokoll von Invitrogen) wurden mit Einzelkolonien des Stammes *Pichia pastoris* GS115pHILDPaHNL5alpha_L1Q,A111G beimpft und für 36 Stunden bei 28 °C geschüttelt (120 rpm). Die Chemikalien 1-9, bemessen für 20 Liter, wurden mit entionisiertem Wasser auf ein Gesamtgewicht von 15 kg gebracht und im 40 L Bioreaktor (MBR, Oftringen, CH) vorgelegt. Nach in situ Sterilisation wurde der pH Wert des Mediums mit 28% Ammoniak über eine sterile Zulaufpumpe auf pH 5.0 eingestellt. Danach wurden 200 ml steril filtrierter "Spurenelement - Lösung" (zusammen mit Vitamin H- Biotin) über eine Zulaufflasche in den Bioreaktor eingebracht. Weitere 200 ml der "Spurenelemente - Lösung" wurden auch jeden zweiten Tag bis Fermentationsende zugegeben. Die Inokulation erfolgte mit 1,4 kg Vorkultur aus den Schüttelkolben. Das Startgewicht des Fermenterinhaltes lag bei ca. 15 kg. Bei einer Arbeitstemperatur von 28 °C, einer Belüftung von 10 - 30 Liter Luft/min und einer Rührerdrehzahl zwischen 350 und 700 rpm wurde der Partialdruck von Sauerstoff (pO2) auf einem Wert > 10 % der Sättigungskonzentration gehalten. Nach 27 Stunden war die Biomasse auf einen Wert von 122,8 g/L Nasszellgewicht bzw. von 30 g/L Zelltrockengewicht (ZTG) angewachsen. Ab diesem Zeitpunkt wurde pro Stunde ca. 70 g steriles Glycerin in kleinen Portionen zudosiert. In dieser linearen Wachstumsphase konnte in einem Zeitraum von 60 Stunden eine Biomassekonzentration im Bereich von 100 g/L ZTG erreicht werden.

Danach wurde die dritte Phase mit der Induktion der Expression durch Zudosierung von Methanol eingeleitet. Dabei wurde der Methanolgehalt in der Kulturbrühe auf einen Wert von 0,8-1 Gewicht % eingestellt. Bei steigendem Sauerstoffverbrauch im Laufe der Fermentation wurde jeweils erneut Methanol (0,8-1 Gewichtsprozent) zugegeben. Der Anstieg der Enzymaktivität wurde durch photometrische Aktivitätsbestimmung im Kulturüberstand von Proben, die aus dem Fermenter ca. alle 12 Stunden entnommen wurden verfolgt. Nach 210 Stunden Induktion mit Methanol war die Zunahme an Enzymaktivität nur mehr sehr gering und die Zellen wurden durch zweimalige Zentrifugation bei 4000 g für 20 min abgeerntet und der Kulturüberstand gesammelt. Die Enzymaktivität im Kulturüberstand betrug nach der Zentrifugation 3,3 U/ml (Standard HNL Assay mit rac. Mandelonitril), was bei einer Gesamtausbeute von ca. 6,5 Liter Kulturüberstand von 14,3 kg Fermenterinhalt eine Enzymausbeute von ca. 22.000 U ergab.

Durch 0,2 µ Crossflowfiltration (VIVASCIENCE Vivaflow 50 von Sartorius, Göttingen, D) wurde der Überstand von restlichem Zellmaterial gereinigt. Die Konzentrierung erfolgte über Crossflow Ultrafiltration mit 30 kDa 50 cm2 Grössenausschlussmodufen von Sartorius. So wurden Enzympräparate mit 24,5 U/ml und 57 U/ml für Pilotversuche zur Cyanhdrinsynthese hergestellt. Da *Pichia pastoris* kaum eigene Proteine in den Kulturüberstand sekretiert, war das so produzierte und aufkonzentrierte Enzym im Vergleich zu pflanzlichen Enzympräparaten auch schon sehr rein.

Folgende Chemikalien wurden zur Herstellung des Kulturmediums benutzt (Menge pro Liter):

| | |
|---|---|
| 1. 85% ortho-Phosphorsäure | 35ml |
| 2. CaSO₄ | 0,68 g |
| 3. K₂SO₄ | 18,8g |
| 4. MgSO₄.7H₂O | 13,4 g |
| 5. KOH | 5,7g |
| (Chemikalien 1 bis 5 in p.a. Qualität) | |
| 6. Glycerin, technische Qualität. 50 ml | |
| 7. Entionisiertes Wasser, Leitfähigkeit 5,5-9,1 µS/cm | |
| 8. Antischaummittel 10 % Acepol 83E (Carl Becker Chemie GmbH, Hamburg, D) 1ml | |
| 9. 25%Ammoniak, technische Qualität 70 g/l | |

Spurenelemente und Vitamin H (alle Chemikalien in p.a. Qualität) :

| | |
|---|---|
| 10. Biotin | 0,8 mg |
| 11. CuSO₄.5H₂O. | 24,0 mg |
| 12. KI | 0,32 mg |
| 13.MnSO₄.H₂O. | 12,0 mg |
| 14. Na₂MoO₄.2 H₂O | 0,2 mg |
| 15.H₃BO₃ | 0,08 mg |
| 16. CoCl₂ | 2,0 mg |
| 17. ZnSO₄.7H₂O | 80 mg |
| 18. Fe(II)SO₄.7H₂O | 260 mg |

### Beispiel 8: Präparative Umsetzungen mit Benzaldehyd und 2-Chlorbenzaldehyd:

Zur Analyse der Enzymeigenschaften in der präparativen Synthese wurden 150mMol Substrat in einem Reaktor umgesetzt.

150 mM Substrat wurden mit 21 mL MTBE verdünnt oder gelöst. 5 mg Enzym "PaHNL5alpha_L1Q,A111G" (A111G Mutante) wurden mit 50 mM K2HPO4/Citrat, pH 3,4 g zu einem Volumen von 37,5mL verdünnt und mit 10%iger Zitronensäure auf pH 3,4 eingestellt. Diese wässrige Phase wurde zur organische Phase chargiert und 5 min in einem 100 mL Schmizo KPG Rührer gerührt. Die Temperatur wurde auf 10°C gehalten und HCN mittels einer Perfuserpumpe für 1 Stunde zudosiert. Die Reaktion wurde bei 10°C bei 900 rpm gerührt. Zur Aufarbeitung wurde die Reaktionslösung mit 140 mL MTBE verdünnt, 5 min gerührt und nach 10 min wurden die Phasen getrennt. Die wässrige Phase wurde nochmals mit 40 mL MTBE extrahiert. Nach spontaner Phasentrennung wurden die organischen Phasen vereint, mit Zitronensäure stabilisiert und einrotiert. Die Analyse mittels GC wurde wie oben beschrieben durchgeführt.

Die Umsetzungen gaben nach 7 Stunden eine Ausbeute von 95,1% 2-Chlorbenzaldehydcyanhydrin mit einem ee von 95,7% bzw. eine Ausbeute von mehr als 99% Mandelonitril mit einem ee >99%.

### Beispiel 9: Enzymstabilität bei tiefem pH

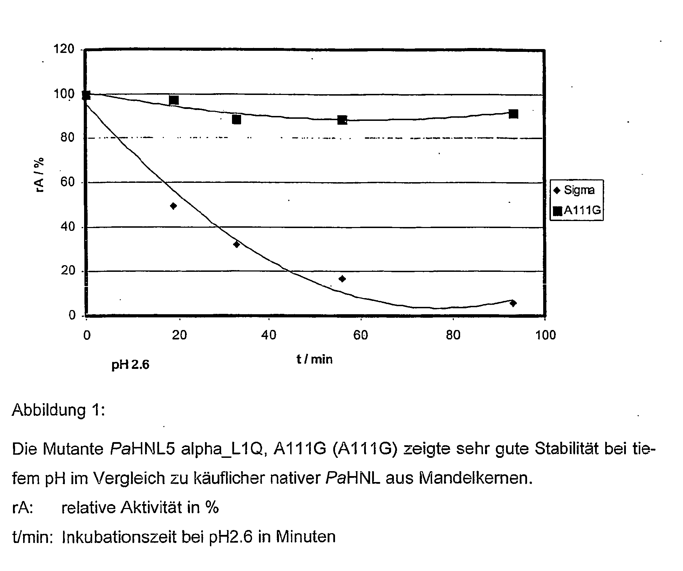

Die Enzymproben von käuflicher nativer *Pa*HNL aus Mandelkernen (Sigma) und der A111G-Mutante wurden in 50mM Citrat-Phosphat Puffer pH 6,5 soweit verdünnt, dass nach einer weiteren 1:70 Verdünnung eine Steigung von ca. 100 mOD bei der photometrischen Aktivitätsbestimmung bei 280 nm nach dem Standard HNL-assay mit racemischem Mandelonitril zu erwarten war. Von diesen Verdünnungen wurden 150µL in 900µL 0.1 M Phosphat-Puffer mit entsprechend eingestelltem pH-Wert überführt (Verdünnung 1:7) und nach Inkubation bei 22°C zu verschiedenen Zeitpunkten wurden dann 100µL dieser Verdünnungen für die Aktivitätsbestimmung eingesetzt (100µL Enzymlösung, 700µL 1M Phosphat-Zitratpuffer pH 5.0 und 200µL 60mM Mandelonitril in 3mM Citrat-Phosphatpuffer pH 3,5). Die pH-Stabilität der Mutante *Pa*HNL5 alpha_L1Q, A111G (A111G) bei pH 2.6 im Vergleich zu käuflicher nativer *Pa*HNL aus Mandelkernen (Sigma) ist aus Abbildung 1 ersichtlich.

## Patentansprüche

1. R-Hydroxynitrillyasen aus Prunus amygdalus oder Prunus serotina **dadurch gekennzeichnet dass** eine erhöhte Substratakzeptanz und erhöhte Stabilität durch die Substitution eines Alaninrests durch einen Glycinrest an einer Position entsprechend Position 111 in der reifen unveränderten rekombinanten R Hydroxynitrillyase PaHNL5 Sequenz erreicht wird.

2. R-Hydroxynitrillyasen nach Anspruch 1, herstellbar durch ortsspezifische Mutagenese.

3. R-Hydroxynitrillyasen, nach Anspruch 2, **dadurch gekennzeichnet, dass** die Herstellung durch ortsspezifische Mutagenese in einem Mikroorganismus ausgewählt aus der Gruppe bestehend aus Pichia pastoris, Saccharomyces cerevisiae, Escherichia coli, Bacilius subtilis, Klyveromyces lactis, Aspergillus niger, Penicillium chrysogenum, Pichia methanolica, Pichia polymorpha, Phichia anomala, und Schizosaccharomyces pombe erfolgt.

4. Verwendung von R-Hydroxynitrillyasen, nach einem der Ansprüche 1-3 zur Herstellung von enantiomerenreinen R-oder S-Cyanhydrinen.

5. Verfahren zur Herstellung von enantiomerenreinen R-oder S-Cyanhydrinen, **dadurch gekennzeichnet, dass** aliphatische, aromatische oder heteroaromatische Aldehyde und/oder Ketone mit R-Hydroxynitrillyasen gemäss einem der Ansprüche 1-3 im organischen, wässrigen oder 2-Phasensystem oder in Emulsion oder in Substanz bei einer Temperatur von -10°C, bis +50°C und einem pH-Wert von 1.8 bis 7 in Anwesenheit eines Cyanidgruppendonors umgesetzt werden.

## Claims

1. R-Hydroxynitrile lyases from Prunus amygdalus or Prunus serotina, **characterized in that** increased substrate acceptance and increased stability is achieved by the replacement of an alanine residue by a glycine residue at a position corresponding to position 111 in the mature unmodified recombinant R-hydroxynitrile lyase paHNL5 sequence.

2. R-Hydroxynitrile lyases according to claim 1, which can be prepared by site-specific mutagenesis.

3. R-Hydroxynitrile lyases according to claim 2, **characterized in that** preparation takes place by site-specific mutagenesis in a microorganism selected from the group consisting of Pichia pastoris, Saccharomyces cerevisiae, Escherichia coli, Bacillus subtilis, Kluyveromyces lactis, Aspergillus niger, Penicillium chrysogenum, Pichia methanolica, Pichia polymorpha, Pichia anomala, and Schizosaccharomyces pombe.

4. Use of R-Hydroxynitrile lyases according to any of claims 1-3 for preparing enantiopure R- or S-cyanohydrins.

5. Process for preparing enantiopure R- or S-cyanohydrins, **characterized in that** aliphatic, aromatic or heteroaromatic aldehydes and/or ketones are converted with R-hydroxynitrile lyases according to any of claims 1-3 in an organic, aqueous or 2-phase system or in emulsion or undiluted at a temperature of from -10°C to +50°C and at a pH of from 1.8 to 7 in the presence of a cyanide group donor.

## Revendications

1. R-hydroxynitrile lyases de *Prunus amygdalus* ou *Prunus serotina*, **caractérisée en ce qu'**on parvient à une acceptation accrue de substrat et à une stabilité accrue par le remplacement d'un résidu alanine par un résidu glycine en une position correspondant à la position 111 dans la séquence de la R-hydroxynitrile lyase recombinante mature non modifiée PaHNL5.

2. R-hydroxynitrile-lyases selon la revendication 1, pouvant être produites par mutagenèse spécifique de site.

3. R-hydroxynitrile lyases selon la revendication 2, **caractérisées en ce que** la production par mutagenèse spécifique de site s'effectue dans un micro-organisme choisi dans le groupe constitué par *Pichia pastoris, Saccharomyces cerevisiae, Escherichia coli, Bacillus subtilis, Kluyveromyces lactis, Aspergilles niger, Penicillium chrysogenum, Pichia methanolica, Pichia polymorpha, Pichia anormala et Schizosaccharomyces pombe*.

4. Utilisation de R-hydroxynitrile lyases selon l'une quelconque des revendications 1-3, pour la préparation de R- ou S-cyanhydrines sous forme d'énantiomères purs.

5. Procédé pour la préparation de R- ou S-cyanhydrines sous forme d'énantiomères purs, **caractérisé en ce qu'**on fait réagir des aldéhydes et/ou des cétones aliphatiques, aromatique ou hétéroaromatiques avec des R-hydroxynitrile lyases selon l'une quelconque des revendications 1-3 en système organique, aqueux ou biphasique ou en émulsion ou en masse, à une température de -10 °C à +50 °C et à un pH de 1,8 à 7, en présence d'un donneur de groupes cyanure.
